# EUROPEAN PATENT APPLICATION

(11) **EP 1 972 350 A1**
(43) Date of publication of application: **24.09.2008**
(21) Application number: 07104509.0
(22) Date of filing: 20.03.2007
(51) Int. Cl.: A61K 47/48

(54) **Dual targeting system**

(71) Applicant: Rijksuniversiteit Groningen, 9712 CP Groningen (NL)
(72) Inventor: The designation of the inventor has not yet been filed
(74) Representative: van Loon, C.J.J.

(57) **Abstract**

The invention relates to the field of drug delivery. In particular, it relates to use of probiotic bacteria, generally Gram-positive bacteria such as *Lactococcus* sp., in providing biologically active polypeptides in the body, especially in the gastrointestinal (GI) tract. Provided is a non-invasive microbial host cell expressing a therapeutic proteinaceous substance, said substance comprising at least one targeting moiety capable of binding to a target cell surface molecule and at least one bioactive polypeptide, wherein said bioactive polypeptide is flanked on both sides by at least one cleavage site for an endopeptidase activity associated with said target cell. Also provided are pharmaceutical and nutritional compositions comprising a host cell according to the invention.

## Description

The invention relates to the field of drug delivery. In particular, it relates to use of micro-organisms, such as probiotic bacteria in providing biologically active polypeptides in the body, especially in the gastrointestinal (GI) tract. In one aspect it relates to target cell specific delivery of therapeutic proteins. Proteinaceous substances, nucleic acid constructs and microbial host cells for these applications are also provided.

Due to technological advances the insight into the molecular basis of disease is rapidly expanding. As a consequence, a multitude of possible therapeutic proteins, including cytokines and signalling molecules, have been identified. The therapeutic use of these possible clinically relevant proteins is often hampered by functional and/or economical considerations. The most important of these considerations are delivery and production of these proteins. Conventionally, therapeutic proteins are purified as to become endotoxin free, which is very expensive, and subsequently systemically administered with a suitable carrier into the bloodstream. The obvious drawbacks of this conventional strategy are unwanted side effects as a consequence of the systemic delivery, as well as (often prohibitively) high production costs.

Very recently, it has emerged that barriers in this respect can be circumvented by using genetically engineered bacteria. Genetically Modified (GM) bacteria that are qualified with high experience to co-exist with humans to produce human therapeutic proteins are opening new ways for those who are suffering from disease conditions: the delivery of proteins in the tractus circumvents the need for endotoxin free preparations, by their very nature bacteria are cheap producers of recombinant proteins and for digestive tract related diseases delivery can be topical rather as systemic.

Microbes have been consumed from ages in a variety of fermented foods and drinks that reside in the intestinal milieu to benefit the host, and these probiotic microbes are classified as GRAS (Generally Regarded As Safe) organisms. Furthermore, these food grade organisms have been genetically engineered to produce various foreign proteins for immune modulation. For example, antigens from pathogens as a prophylactic immunization, and proteins like cytokines, interleukins, and interferons have been expressed.

It is known in the art that local delivery of therapeutic proteins to the mucosa can be easily achieved by administering GM bacteria that produce therapeutic proteins. For example, UK patent GB2278358B discloses a system for the expression of heterologous antigens in the non-pathogenic, non-colonising, non-invasive food-grade bacterium *Lactococcus lactis.* GB-2278358B shows that heterologous antigen can be fully antigenic when accumulated within the cytoplasm of *L. Lactis,* from which it is presumed to leak in vivo as the cells are digested by phagocytic cells. Also it has been shown that *L. lactis* is able to produce and secrete biologically active murine IL-2 when cultured *in vitro* (Steidler et al., Applied and Environmental Microbiology, April 1995, Vol. 61, No. 4,pp1627-1629). EP0871748 relates to nucleic acid constructs for co-expression in *L. lactis* of an antigenic polypeptide and a biologically active cytokine polypeptide. Braat *et al.* conducted the first successful human trial with a GM *Lactococcus lactis* in which the thymidylate synthase gene was replaced with a synthetic sequence encoding mature human IL-10 (Clin. Gastroenterol.Hepatol. (2006), 4:754-759).

The GI tract is the locus for many different pathologies, including cancer and inflammatory disease, whose treatment remains problematic. Although substantial insight has been gained in the pathogenesis of these diseases (Braat et al., 2006a;Vogelstein and Kinzler, 2004), translating this knowledge to novel therapy remains troublesome. Clearly, the above indicates that GM non-invasive bacteria may be a powerful tool for the local delivery of bioactive proteins in the GI tract. However, a major disadvantage of GM bacteria used thus far has not allowed any further targeting of powerful recombinant proteins within the intestinal tract, for example a specific targeting to diseased cells. Potentially, a pleiotropic action of bioactive molecules could lead to unwanted side effects. Also, the delivery of functional protein therapeutics to the mucosa of the digestive tract has in some cases proven to be difficult mainly as a consequence of the protein-structure hostile nature of the lumen of the tract.

The inventors therefore set out to develop a system based on transgenic microbes that avoids one or more of the above drawbacks. In particular, it is a goal of the invention to provide an advanced level of control of recombinant protein delivery and action. A further goal is the provision of a targeting system wherein a bioactive polypeptide is released by a GM micro-organism at a diseased target cell, for example colon cancer cells or inflammatory intestinal epithelial cells. This would open up the road for the (clinical) use of potent bioactive polypeptides, which would otherwise be hampered because of their pleiotropic effects and/or loss of functionality.

Based upon new insights, the present inventors realised that one or more of the above goals can be met by the provision of a "dual targeting system", which exploits the endogenous protease activity of a target cell to specifically release a functional bioactive molecule at the site of the target cell. In this dual targeting system, a GM micro-organism, e.g. a probiotic bacterium, expresses at its surface a recombinant fusion protein comprising a bioactive polypeptide, wherein the polypeptide is flanked on both sides by at least one cleavage site for an endopeptidase of a target cell, for instance an epithelial endopeptidase. Furthermore, the fusion protein comprises a binding moiety capable of recognizing and binding to a cell surface antigen expressed by the target cell. Upon delivery of the GM probiotic to the target cell by virtue of the binding moiety, the endopeptidase activity of the target cell cleaves the cleavage sites flanking the bioactive polypeptide, thereby releasing it from the membrane of the probiotic to exert a local effect. Since the release of the bioactive polypeptide is dependent on a protease activity of the targeted cell, an enhanced level of control of the site of delivery can be obtained.

Accordingly, the invention relates to a therapeutic proteinaceous substance comprising at least one targeting moiety capable of binding to a target cell surface molecule and at least one bioactive molecule, wherein said bioactive molecule is flanked on both sides by at least one cleavage site for an endopeptidase activity associated with said target cell. Preferably, the enzyme activity is localized extracellularly of said target cell such that it can easily access the therapeutic substance once brought in close proximity of the target cell by the action of the at least one targeting moiety. It is for instance an endopeptidase activity bound or associated with the apical membrane of a polarized epithelial cell.

The at least one targeting moiety can be any type of proteinaceous structure capable of binding to a cell surface molecule expressed on a target cell. In a preferred embodiment, it is an antibody or a functional fragment thereof. Recombinant antibodies have an increasing number of applications in biotechnology and medicine. The molecular techniques allow to generate a recombinant antibody which maintains the antigen-binding competence of the parental monoclonal antibody. The antigen binding ability of the antibody is usually conserved when only the V_{H} and V_{L} domains are connected with a polypeptide linker to constitute a single chain variable fragment (scFv) recombinant antibody. The scFv recombinant antibody contains the complete antigen-binding site of an antibody. The smaller format of the scFv (27kDa) allows significant advantages of formatting (fusion with others molecules) and manufacturing in a recombinant host cell, for example in a bacterial production system.

In a particular aspect of the invention, the at least one targeting moiety, allows for specific or at least increased targeting the proteinaceous substance comprising at least one bioactive polypeptide to a diseased cell. Also contemplated is a proteinaceous substance comprising two or more targeting moieties. This allows for enhanced targeting specificity.

The at least one targeting moiety targets for example a tumor cell or a cell that is inflamed or otherwise stressed. This can be achieved by the design and/or selection of at least one targeting moiety, e.g. a scFv, capable of binding to a target cell surface molecule selected from the group consisting of ligands that are overexpressed or exclusively expressed on diseased cells, including tumor cells or otherwise aberrant (e.g. inflamed) cells, such as tumor-specific antigens and stress induced proteins. In one aspect of the invention, the targeting moiety allows for cancer-cell selective targeting of a bioactive polypeptide. This can be achieved by choosing a targeting moiety which is reactive with a surface molecule that is overexpressed on tumor cells as compared to non-tumor cells. Suitable surface molecules that can be targeted to achieve cancer-cell selective targeting include the Epidermal growth factor receptor 3 (EGF-R3) and EGP-2. EGP-2 is of particular interest for targeting an epithelial tumor cell.

In one aspect, the at least targeting moiety allows for targeting of inflamed tissue, including intestinal inflamed tissue. For example, the targeting moiety is capable of recognizing and binding to a glycoprotein encoded by a nonclassical MHC class I chain-related (MIC) gene MICA or MICB. The highly polymorphic nonclassical MHC class I chain-related (MIC) genes MICA and MICB encode stress inducible glycoproteins expressed on a variety of epithelial cells, including intestinal cells. Interestingly, the MIC genes have been reported to be attractive functional and positional candidate genes for inflammatory bowel disease susceptibility as a consequence of their position in the HLA region and expression on the gastrointestinal epithelium.

A targeting moiety specific for essentially any antigen, be it a peptide, sugar, lipid, nucleic acid or whole organism etc., can be selected by methods known in the art. Peptide libraries containing large amounts of randomly synthesized peptides which can be used in selecting a suitable binding partner for an antigen of interest are commercially available. For instance, New England Biolabs offers pre-made random peptide libraries, as well as the cloning vector M13KE for construction of custom libraries. The pre-made libraries consist of linear heptapeptide and dodecapeptide libraries, as well as a disulfide-constrained heptapeptide library. The randomized segment of the disulfide-constrained heptapeptide is flanked by a pair of cysteine residues, which are oxidized during phage assembly to a disulfide linkage, resulting in the displayed peptides being presented to the target as loops. All of the libraries have complexities in excess of 2 billion independent clones. The randomized peptide sequences in all three libraries are expressed at the N-terminus of the minor coat protein pIII, resulting in a valency of 5 copies of the displayed peptide per virion. All of the libraries contain a short linker sequence (Gly-Gly-Gly-Ser) between the displayed peptide and pIII.

Of particular interest is the use of phage display technology. Many reviews on phage display are available, see for example Smith and Petrenko [1997] Chem. Rev. 97:391-410. Briefly, phage display technology is a selection technique in which a library of variants of a peptide or human single-chain Fv antibody is expressed on the outside of a phage virion, while the genetic material encoding each variant resides on the inside. This creates a physical linkage between each variant protein sequence and the DNA encoding it, which allows rapid partitioning based on binding affinity to a given target molecule (antibodies, enzymes, cell-surface receptors, etc.) by an in vitro selection process called panning. In its simplest form, panning is carried out by incubating a library of phage-displayed peptides with a plate (or bead) coated with the target (i.e. antigen of interest), washing away the unbound phage, and eluting the specifically bound phage. The eluted phage is then amplified and taken through additional binding/amplification cycles to enrich the pool in favour of binding sequences. After 3-4 rounds, individual clones are typically characterized by DNA sequencing and ELISA. The DNA contained within the desired phage encoding the particular peptide sequence can then be used as nucleic acid encoding a targeting means for use in the present invention. Alternatively, high-affinity binders can be selected from combinatorial libraries of designed ankyrin repeat proteins (Binz et al. Nat. Biotechnol. 2004; 22(5):575-82. Also suitable for use as cell targeting means are iMabs (industrial Molecular affinity bodies) developed by CatchMabs BV, Wageningen, The Netherlands. IMabs are small designer proteins that can bind target molecules with both high affinity and high specificity, even under the harsh chemical conditions of the processing industry. A general overview of technology related to the design of a specific targeting moiety can be found in Binz and Pluckthun (Curr Opin Biotechnol. 2005 Aug;16(4):459-69).

Still a further possibility is the selection of appropriate V_{H} and V_{L} genes from antibody producing cells, such as monoclonal antibody producing hybridoma cells or by direct cloning from relevant human Ig producing cells

The target cell can be any type of target cell of interest, provided that it produces a protease capable of releasing the at least one bioactive polypeptide contained in a proteinaceous substance of the invention. It can be a mammalian cell, preferably a human cell, or a bacterial cell. More preferably, it is an epithelial cell, in particular an intestinal epithelial cell. These cells are particularly good accessible by a probiotic producing and expressing a proteinaceous substance of the invention.

Accordingly, in one embodiment, the invention provides for a therapeutic proteinaceous substance comprising at least one targeting moiety, said targeting moiety being capable of binding to a cell surface molecule of an intestinal epithelial cell, and at least one bioactive molecule, wherein said bioactive molecule is flanked on both sides by at least one cleavage site for an epithelial endopeptidase.

Intestinal epithelial cells express the Poly immunoglobulin receptor (pIgR) (Brandtzaeg et al., 1999). The pIgR serves to transport polymeric immunoglobulins (pIgs) produced by plasma cells across the epithelial cells in order to exert their protective effects against environmental antigens. For example, dimeric IgA binds to the pIgR present on epithelial cells of the mucosa and is subsequently transported to the lumen of the gut. Then the IgA is released into the gut lumen together with the extracellular part of the pIgR as a protein complex known as secretory IgA (SIgA) (Johansen and Brandtzaeg, 2004). pIgR is composed of six domains, the 6th domain containing the protease cleavage site (Asano et al. Immunology 2004, 112;583-589), The inventors realised that this cleavage site on the pIgR can be exploited for specific drug delivery because epithelial cells have protease activity capable of specifically hydrolyzing and cleaving this site in the complex. Therefore, in a specific aspect the invention provides a therapeutic proteinaceous substance comprising at least one targeting moiety, said targeting moiety being capable of binding to a cell surface molecule of an intestinal epithelial cell, and at least one bioactive molecule, wherein said bioactive molecule is flanked on both sides by at least one cleavage site found in pIgR. Preferably, the cleavage site corresponds to a cleavage site that is found in domain 6 (region from Tyr553 to Arg627) of the human PolyIgR. It will of course be understood that only a stretch of amino acids is present that determines protease susceptibility. This is not necessarily the whole domain 6. Furthermore, one or more amino acid residues of a cleavage site found in wildtype pIgR may be altered, e.g. by a conservative amino acid substitution, as long as its protease sensitivity is maintained. Protease sensitivity can be determined in vivo or in vitro. In vitro testing is of course preferred. For example, a proteinaceous substance comprising a one or more potential cleavage sites for an epithelial (or any other type of) target cell is prepared as recombinant protein and subsequently contacted with said epithelial cell. Thereafter, it is determined whether or not the recombinant protein is cleaved at the desired site(s), e.g. by SDS-PAGE analysis, optionally followed by Western blotting. For instance, release of bioactive polypeptide flanked by one or more potential cleavage sites is measured upon incubation with a target cell culture. Cleavage of the recombinant protein is indicative of the cleavage site being suitable for use in a proteinaceous substance according to the invention. As will be understood by the skilled person, positive and negative control proteins are preferably included and are more preferably tested in parallel.

As indicated herein above, it would be beneficial for the use of a therapeutic proteinaceous substance in the GI tract, e.g. by using non-invasive or non-pathogenic bacteria to deliver it, that the proteinaceous substance be protected against the hostile environment. This would increase the chances that the bioactive polypeptide is delivered in an intact form at its intended site of action, for instance at a diseased intestinal epithelial cell and it would increase the half-life of the therapeutic proteinaceous substance after its release from the micro-organism..

Accordingly, the proteinaceous substance comprises in addition to the at least one cell targeting moiety and the at least one bioactive polypeptide flanked by protease cleavage sites, at least one stabilizing domain conferring stability of the substance in the gut. The inventors realised that molecules that normally reside in the GI tract should be able to withstand protein degradation and therefore can be exploited as stabilizing domain in a proteinaceous substance of the invention. The principal antibody on most mucosal surfaces is Secretory Immunoglobulin A (SIgA). It is a polypeptide complex consisting of dimeric IgA, the linking J chain and the secretory component. Its molecular stability and its in general anti-inflammatory properties make SIgA, or part thereof, not only an ideal molecule to function as stabilizing domain. However, a related structure such as an intact IgG constant region (IgG Fc) or an IgA constant region (IgA Fc) may also suitably used as stabilizing domain.

In one embodiment the invention therefore provides a therapeutic proteinaceous substance comprising at least one targeting moiety capable of binding to a target cell surface molecule and at least one bioactive molecule, wherein said bioactive molecule is flanked on both sides by at least one cleavage sites and wherein the substance furthermore comprises at least one stabilizing domain selected from an IgG constant region (IgG Fc) or an IgA constant region (IgA Fc), preferably IgA Fc. Particularly preferred is SIgA consisting of IgA Fc complexed to domains 1-5 of the human polyIgR (also known as SC2)

The above describes and explains the various options to achieve a dual targeting system for the controlled delivery and release of one or more bioactive polypeptides at a specific target site. The term "bioactive" refers to the polypeptide having a biological activity when released from the proteinaceous substance. It may or it may not be active before cleavage, i.e. when being part of the proteinaceous substance. The term "therapeutic" is meant to indicate that the bioactive polypeptide finds its use in (prophylactic) therapy. It will be understood that the dual targeting system can be used for the delivery of any type of proteinaceous molecule, or combination of two or more bioactive polypeptides. For therapeutic purposes, it is contemplated that the least one bioactive polypeptide affects the viability, growth and/or differentiation of target cells. It may be selected from the group consisting of cytokines, growth factors, antigens, differentiation factors, morphogenic polypeptides, apoptosis-inducing polypeptides and IgA and IgG antibodies. The bioactive polypeptide may modulate the expression or production of a substance by a target cell. Exemplary bioactive polypeptides include bone morphogenetic protein (BMP), hedgehog, TGF-β family members, TNF-α, IFNγ, ligands for death receptors such as sTRIAL and FasL. It can also be an immunogenic polypeptide.

A proteinaceous substance of the invention may comprise multiple copies of one type of bioactive polypeptides, each flanked by one or more cleavage sites. Alternatively, or in addition, multiple types of bioactive polypeptides may be present within a single proteinaceous substance.

The relative orientation, i.e. from N- to C-terminus, of the different functionalities within a proteinaceous substance of the invention can vary. In case VH and VL fragments are used as targeting moieties, they are typically placed directly adjacent to each other via a linker sequence. The constant IgA or IgG part is preferably placed in close proximity to an SC2 functionality. The at least one bioactive polypeptide can be positioned either N- or C-terminal from the targeting moiety. However, to minimize sterical hindrance between targeting moiety and target cell surface molecule and maximize target cell recognition, it is preferred that the at least one targeting moiety is good accessible.
In one embodiment, a proteinaceous substance of the invention comprises, from N- to C-terminus, the following functionalities: membrane anchoring domain- cleavage site-bioactive polypeptide- targeting means, for example MleP-SC1-BMP2-SC1-V_{L}-V_{H.}-In another embodiment, a proteinaceous substance of the invention comprises, from N-to C-terminus, the following functionalities: membrane anchoring domain- cleavage site- bioactive polypeptide- targeting means- stabilizing domain, for example MleP-SC1-BMP2-SC1-V_{L}-V_{H}-C-alpha-SC2.

A further aspect of the invention relates to an nucleic acid sequence encoding a proteinaceous substance as provided herein. The skilled person will be able to design and construct such a nucleic acid sequence. Likewise, the invention provides an expression vector comprising at least one nucleic acid sequence encoding a proteinaceous substance of the invention. The vector can be introduced into a host cell, allowing for expression of the protein encoded. Suitable vectors are known in the art. For example, the nucleic acid sequence is cloned into a vector suitable for expression in a bacterial cell. Suitable vectors comprising nucleic acid for introduction into bacteria can be chosen or constructed, containing appropriate regulatory sequences, including promoter sequences, terminator fragments, enhancer sequences, marker genes and other sequences as appropriate. Vectors may be plasmids, viral e.g. 'phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Short Protocols in Molecular Biology, Second Edition, Ausubel et al. eds., John Wiley & Sons, 1992.

Prior art disclosures have primarily focused on the use of GM bacteria to produce and secrete a bioactive polypeptide. To that end, a nucleic acid encoding the biologically active polypeptide comprises a secretory signal sequence providing for secretion of the biologically active peptide. This is in contrast to the present invention, wherein the proteinaceous substance is preferably remains bound to the bacterial host cell by which it is produced until a) the host cell-protein complex reaches (by virtue of the targeting moiety) a desired target cell and b) a protease activity of said target releases the bioactive polypeptide from the proteinaceous substance.

To achieve expression of a proteinaceous substance on the surface of a microbial host cell, the at least one nucleic sequence encoding said substance is cloned in frame with a sequence encoding a membrane anchoring domain, for example an endogenous or heterologous transmembrane protein. Cell surface expression of a proteinaceous substance of the invention on a lactic acid producing bacterium may involve the use of the membrane potential-generating malate (MleP) transporter of lactic acid bacteria. The mleP sequence is available under accession number X75982 (Bandell et al. J Biol Chem. 1997; 272(29):18140). As is disclosed herein, plasmid pNZmleP described previously (Biochemistry. 2000;39(42):13059) may be suitably used to practise the present invention. Preferably, a nucleic acid sequence encoding a proteinaceous substance is ligated at the 3'-terminal end of and in frame with the mleP gene such that the resulting protein comprises a proteinaceous substance fused to the C-terminus of MleP.

Other transmembrane proteins that may be used include OpuA, glycine betaine transporter protein. (Van der Heide T and Poolman B, EMBO Reports, vol 3, p 938-943, 2002). The OpuA protein has as advantage that it has an extra-cytoplasmatic substrate binding domain that sticks out into the extra-cellular space. By coupling of a therapeutic proteinaceous substance to the C terminus of the extracellular part of OpuA, or similar tyope of transmembrane protein, the proteinaceous substance will stick out further from the membrane. This can contribute to improved recognition and binding to a target cell. Yet another exemplary membrane anchoring domain is the Gln PQ protein
(Schuurman-Wolters, G.K. and Poolman, B, Journal of Biological Chemistry, vol 280, p23785-23790, 2005). This is also an ATP-binding cassette (ABC) transporter protein that has two extra-cytoplasmatic substrate binding domains. An extra advantage of this protein is that the extra-cytoplasmatic domains are first built in after the signal peptide sequence and then cleaved off to extend to the outside. A nucleic acid sequence encoding a proteinaceous substance of the invention can be cloned in between the signal peptide and the second substrate binding domain. As a resultant, the vector will be reversed in orientation with the cell targeting means sticking out into the extracellular space. Accordingly, in a preferred embodiment the invention provides a nucleic acid construct encoding a therapeutic proteinaceous substance according to the invention, cloned in frame with an ABC transporter. Also provided is the fusion protein encoded by such construct, as well as an expression vector comprising such construct. In particular, there is provided a bacterial host cell provided with the construct, or vector, and wherein the chromosome of said host cell contains the gene encoding the ABC transporter, be it in intact or disrupted form.

One expression vector may comprise more than one nucleic acid sequence encoding a therapeutic proteinaceous substance as provided herein. In a preferred embodiment, the coding sequences for a first biologically active polypeptide and a second biologically active polypeptide are contained in an operon, i.e. a nucleic acid construct for multi-cistronic expression. In an operon, transcription from the promoter results in a mRNA which comprises more than one coding sequence, each with its own suitably positioned ribosome binding site upstream. Thus, more than one polypeptide can be translated from a single mRNA. Use of an operon enables expression of the biologically active polypeptides to be co-ordinated.

A promoter employed in accordance with the present invention can be an inducible or a constitutive promoter. Preferably, the promoter directs expression at a level at which the bacterial host cell remains viable, i.e. retains some metabolic activity, even if growth is not maintained. Use of a constitutive promoter avoids the need to supply an inducer or other regulatory signal for expression to take place. On the other hand, an inducible promoter allows for controlled expression. Suitable inducible promoters include the nisin promoter. In a specific aspect, the endogenous promoter of an endogenous membrane anchoring domain to which the proteinaceous substance is coupled is advantageously used. For instance, it is the promoter of MIeP, GlnPQ or OpuA.
A further aspect of the invention relates to a non-invasive or non-pathogenic microbial host cell provided with at least one expression vector as described above. A host cell may comprise two or more different types of vectors, for instance a first vector encoding a first proteinaceous substance comprising a first biologically active polypeptide and a second vector encoding a second proteinaceous substance comprising a second biologically active polypeptide. The nucleic acid sequences in the separate vectors may be under the regulatory control of separate promoters. The promoters may be the same or different. Transformation of a host cell with one or more vectors according to the invention can be performed using methods known in the art including protoplast transformation and electroporation.

A host cell according to the invention is a non-invasive or non-pathogenic micro-organism. It may be a non-colonising bacterium. Food-grade and probiotic bacteria are especially suitable. In a preferred embodiment it is a Gram-positive bacterium, for example a *Lactococcus* species, *Listeria innocua* or *Staphylococcus xylosus.* Of particular interest are *Lactococcus lactis, Lactobacillus plantarum, Lactobacillus casei,* and *Lactobacillus delbruckei* Alternatively, an attenuated Gram-positive pathogenic bacterium may be used, for example *Listeria monocytogenesis.* Also, yeast cells may be used as delivery vehicle, such as *Saccharomyces cerevisiae.* (Blanquet et al. J Biotechnol. 2004 May 13;110(1):37-49).

As is the case for most, if not any, type of GM organism it will be understood that spreading and surviving of a genetically modified bacterial host cell of the invention is unwanted. To avoid unintentional release of GM organisms, special guidelines for safe handling and technical requirements for physical containments are used. In addition, biological containments measures may however be taken to even further reduce the possibility of survival of the GM organism in the environment. Methods for biological containment are known in the art. See for example EP1383897 and references therein.

Therefore, in one aspect of the invention, the non-invasive or non-pathogenic micro-organism genetically modified to allow for expression of the heterologous proteinaceous substance has an environmentally limited growth and viability. For example, it is genetically modified bacterial host cell which only survives in a medium comprising well-defined medium components.

It may be a bacterial strain. e.g. a *Lactococcus* sp., that is dependent on thymine or thymidine. Such a strain can be constructed by inactivation of a thymidylate synthase (*thyA*) gene using gene disruption, for instance by the nucleic acid construct encoding a proteinaceous substance according to the invention. Inactivation of the *thyA* gene has been proposed as a biological containment tool for microorganisms intended to be released into the environment. The *thyA* gene codes for thymidylate synthase (TS), the enzyme responsible for the catalytic conversion of dUMP into dTTP. Bacterial strains bearing deletions within the *thyA* gene are auxotrophic for thymine or thymidine and are not expected to proliferate in the environment, where free pyrimidines are absent.

Alternatively, a nucleic acid construct of the invention encoding a therapeutic proteinaceous substance fused to a membrane anchoring domain is inserted into the chromosome of the microbial host cell, e.g. by natural recombination or by targeting. By homologous recombination with the native gene for the membrane anchoring domain, such as Mlep, OpuA or GlnPQ, the original gene will be replaced by a recombinant gene. The efficiency for homologous recombination can be very high because of the large stretches of homology between the target gene in the chromosome and the vector sequence. The membrane anchoring protein will maintain its function as a transporter molecule for a specific substrate, such as malate for Mlep. glycine betaine for OpuA, or glutamine and glutamic acid for GlnPQ. By using host bacterial strains that are completely dependent on one or more of these specific substrates, only those bacteria will survive that are dependent on an exogenous source of the substrate (like for thymine for the Thy gene) and on the action of the recombinant transporter molecule. Host cells having a non-functional membrane transporter, and/or those being in an environment lacking the appropriate substrate, will die.

Based on the above, it is clear that a microbial host cell according to the invention is useful for the delivery of therapeutic polypeptide. The invention thus provides a composition, preferably a food or pharmaceutical composition, comprising a non-invasive or non-pathogenic microbial host cell according to the invention and a suitable carrier. Whereas a dual targeting system of the invention is primarily meant to be based on the delivery of a proteinaceous substance by means of a GM organism expressing the substance, a recombinant proteinaceous substance may, in theory, also be used as such as therapeutic agent. It can be produced in various types of (bacterial) recombinant expression systems and incorporated in a food or a pharmaceutical composition.

A further aspect relates to the use of a proteinaceous substance and/or non-invasive or non-pathogenic microbial host cell for the manufacture of a medicament for the treatment or prophylaxis of a disease or disorder. The medicament may be for human use or veterinary use. The design of a dual targeting system of the invention is such that both the targeting moietie(s) and the bioactive polypepeptide(s) can be tailor made for a specific purpose.

The dual targeting system provided herein is of particular interest for the treatment or prevention of a gastrointestinal disease or disorder. Still further, the bioactive polypeptide may be an immunogenic polypeptide, allowing for the manufacture of an oral vaccine.

Gastrointestinal disorders that could benefit from such a medicament include cancer, chronic inflammatory conditions such as inflammatory bowel disease (IBD), and gastrointestinal infections. Also, subjects treated with cytostatic drugs or immunosuppressant (e.g. corticosteroids), such as cancer patients, transplanted or autoimmune patients, who suffer from a depressed mucosal immune system, treated with, may receive the medicament as supportive therapy.

Also provided is a method for preventing or treating a disease, preferably an intestinal disease, comprising administering to a person in need thereof a therapeutically effective dose of a composition of the invention. A method of the invention may also comprise an oral immunization method.
Intestinal diseases that may benefit from therapy include chronic intestinal inflammatory conditions (e.g. inflammatory bowel disease), coeliac disease, polyposis and colon carcinoma. Inflammatory bowel disease (IBD) is a group of inflammatory conditions of the large intestine and, in some cases, the small intestine. The main forms of IBD are Crohn's disease and ulcerative colitis (UC). The main difference between Crohn's disease and UC is the location and nature of the inflammatory changes in the gut. Crohn's can affect any part of the gastrointestinal tract, from mouth to anus (skip lesions), although a majority of the cases start in the terminal ileum. Ulcerative colitis, in contrast, is restricted to the colon and the anus. Other forms of IBD accounting for far fewer cases are Collagenous colitis, Lymphocytic colitis, Ischaemic colitis, Diversion colitis, Behçet's syndrome, Infective colitis, Irritable Bowel syndrome (IBS) and Indeterminated colitis.

Coeliac disease (CD) is a chronic inflammatory intestinal disease with known environmental trigger and a strong genetic component. The external trigger is ingested wheat gluten or related proteins from rye, barley and possibly oats. CD is a multigenic disorder with a strong HLA association. The disease is also known as gluten sensitive enteropathy. It causes considerable morbidity, often presenting with diarrhoea, malnutrition and failure to thrive in infants, or diarrhoea and osteoporosis in later life. Infertility, autoimmune diseases and lymphomas have also been associated with CD. In one embodiment, the invention provides a therapy for the treatment of CD, comprising the delivery of a proteinaceous substance to inflamed intestinal epithelial cells wherein the bioactive polypeptide is IL-10, or an anti-TNFα antibody, and wherein the targeting moiety is capable of binding to EGP-2.

Colon cancer, also called colorectal cancer or bowel cancer, includes cancerous growths in the colon, rectum and appendix. It is the third most common form of cancer and the second leading cause of death among cancers in the Western world. Many colorectal cancers are thought to arise from adenomatous polyps in the colon. These mushroom-like growths are usually benign, but some may develop into cancer over time. The majority of the time, the diagnosis of localized colon cancer is through colonoscopy. Therapy is usually through surgery, which in many cases is followed by chemotherapy. In one embodiment, the invention provides a therapy for the treatment of colon cancer, comprising the delivery of a proteinaceous substance to colon cancer cells by virtue of a cancer specific targeting moiety, for example anti-EGP-2. Local protease-mediated release of an apoptotic polypeptide, such as BMP-2 or an other type of pro-apoptotic molecule, will trigger cancerous cell to undergo programmed cell death. This allows for a specific and early treatment of colon carcinoma.

Familial adenomatous polyposis (FAP) is an inherited condition in which numerous polyps form mainly in the epithelium of the large intestine. While these polyps start out benign, malignant transformation into colon cancer occurs 100% of the time when not treated. The incidence of the mutation is between 1 in 10,000 and 1 in 15,000 births. By age 35 years, 95% of individuals with FAP have polyps. Without colectomy, colon cancer is virtually inevitable.

Treatment for FAP will require frequent surveillance colonoscopy investigations; in a number of cases, removal of the colon is necessary to prevent the development of malignancy, or to cure it. Various medications are being investigated for slowing malignant degeneration of polyps, most prominently the non-steroidal anti-inflammatory drugs (NSAIDs). According to the invention, a dual targeting system is provided for the treatment or prophylaxis of FAP wherein a therapeutic proteinaceous substance comprising a suitable bioactive polypeptide is used, such as Sonic hedgehog, TRAIL or FAS ligand.

### Legends to the Figures

Table-1: Primer list, which was used to amplify different gene to construct scFv SIgA. The restriction enzymes introduced into the vector are underlined and bold.
Table-2: The scFvSIgA was developed from the backbone MOC31IgG. This table contains the number of amino acid of each MOC31 vector, their expected size of proteins. To confirm their expression the proteins was probed with different specific antibodies.
Figure-1: Schematic representation of scFvSIgA construction from MOC31IgG with the plasmid size and proteins prediction with their important properties.
Figure-2: (A) Schematic representation of scFvSIgA expressed on *Lactococcus lactis* membrane protein MleN, (B)) Schematic representation of scFv MOC31SIgA with their respective functions.
Figure-3: COS-7 cells were transfected with MOC31 vectors, followed by staining with anti-human IgA FITC and DAPI.
Figure-4: MOC31 vector transfected COS-7 cell's supernatant was used to analysis their protein production by ELISA.
Figure-5: Analysis of recombinant scFv polypeptide, proteins expression and their identity confirmed by immunodetection using specific antibody. The polyacrylamide with different percentage of SDS-containing gels was prepared. The Mr of the scFv and Ab indicated was calculated from protein markers run in parallel.
Figure-6: Ramos cells were transfected with EGP-2. Supernatants of COS-7 cells which were transfected with different MOC31 vector was incubated with transfected Ramos cells. This was followed by FACS analysis, to show MOC variant retains its binding properties.
Figure 7 : Confirmation of correct insert of BMP-2 into the BMP-DIgAslac vector by PCR. The complete insert consisted of MOC31 heavy and light chain variable parts, specific for the colonic epithelial molecule EGP-2 insert, the complete human IgA constant region, two domains 6 of the human polyIgR containing the epithelial endopeptidase sensitive sites SC1 and SC2 and Bone Morphogenetic Protein-2. The insert was further confirmed by sequencing and expression
Figure 8 :Schematic drawing illustrating the dual targeting concept of the invention.

The invention is illustrated further by the examples below.

### Example 1: Targeting of recombinant scFvSIgA1 protein anchored to L. lactis to colon cancer epitope.

This example describes the construction and evaluation of a novel recombinant scFvSIgA1 protein produced by *L. lactis,* anchored to bacterial membrane, which retains its full immunorecognizing potential. The scFv fragment employed was a sequence targeted to a colon cancer epitope, EGP-2. Accordingly *L. lactis* expressing this chimeric protein was capable of binding cells expressing this epitope.

### Materials and methods.

### Construction of scFvMOC31IgA1/2.

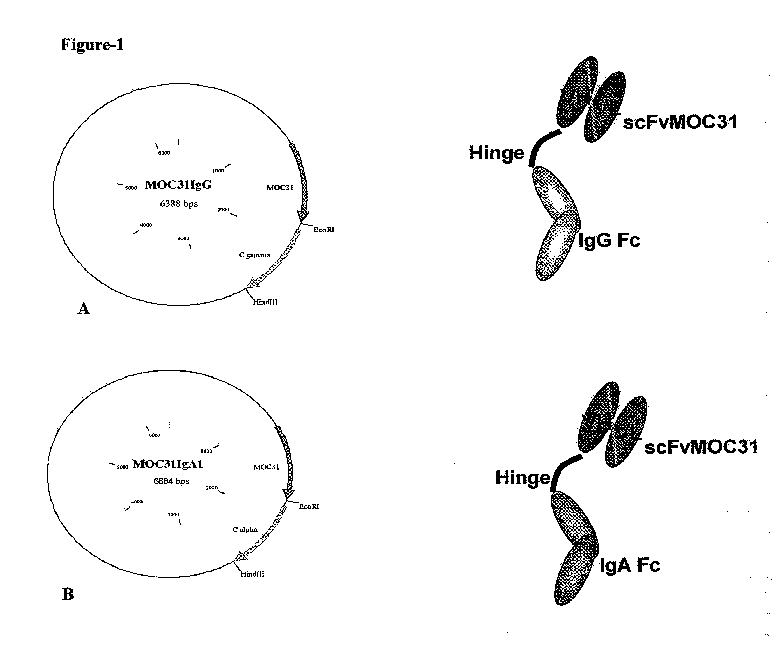

The Cα1/2 genes were amplified from normal ileum of human cDNA with *Eco*RI/*Hind*III restriction sequences (Table-1) using PCR method. Cγ1 region present in MOC31IgG plasmid (Helfrich *et al.,* 1998) was replaced with Cα1 and Cα2 using above mentioned restriction enzymes to obtain MOC31-IgA1 and MOC31-IgA2 constructs, respectively (Figure 1B). Sequence analysis confirmed the correct and in-frame of the pMOC31IgA1/IgA2 (data not shown).

### Construction of scFvMOC31IgA1SC1.

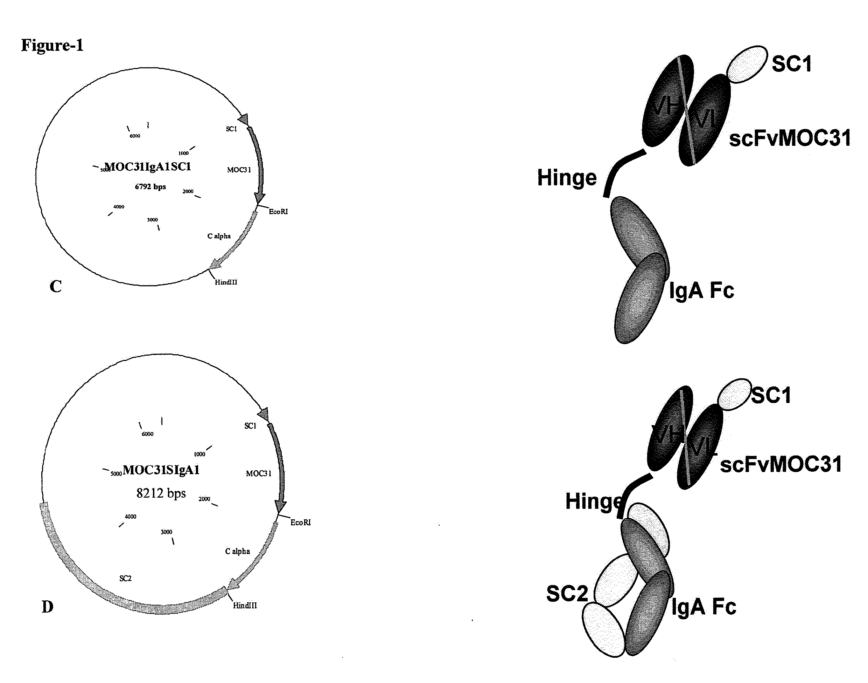

SC1 is the sixth domain of the human PIgR and contains a proteolytic cleavage site. SC1 DNA fragment was amplified from the entire PIgR vector. MOC31SC1IgA1 and MOC31SC1IgA2 were constructed by inserting SC1 using *Bgl*II*lPfo*I restriction enzymes into MOC31-IgA1 and MOC31-IgA2 (Figure 1C).

### SC2

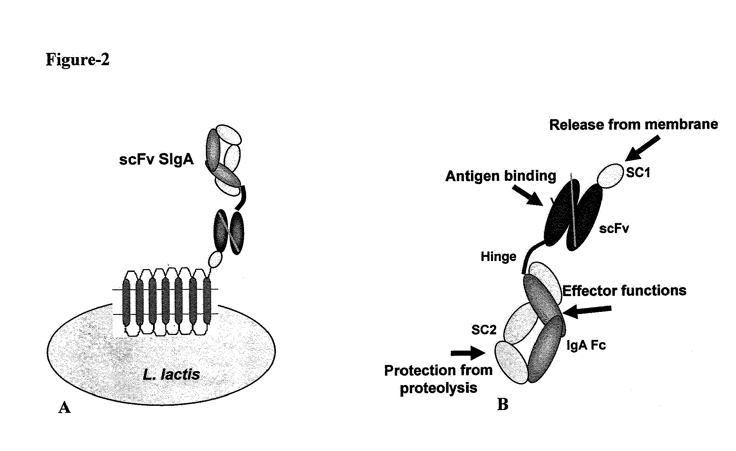

The remaining 5 domains of pIgR (referred as SC2) were amplified from SC-his6 plasmid. For subcloning, 5'*Hind*III and 3' *Van*91I were introduced in SC2. The entire five domains were cloned into the MOC31SC1IgA1 and MOC31SC1IgA2 to get MOC31SIgA1/SIgA2 (Figures 1D and 2A).

### pNZmlePSIgA1

pNZmleP encodes the membrane protein MleP that is a malate transporter protein in *L. lactis.* The pNZ*mle*P was constructed as described in reference ((Bandell and Lolkema, 2000)). To ligate the entire MOC31SIgA1 into the pNZ*mle*p, A*fl*III restriction site was used. Additional A*fl*III restriction site was added at the 3'end of the MOC31SIgA1 by PCR. MOC31SIgA1 was ligated at the C-terminal of the *mlep* gene at the A*fl*IIII restriction site (Table-1). The cells with right orientation plasmid can grow. Because the A*fl*III restriction enzymes digest ACRYGT and we used different nucleotide at the end of the MOC31SIgA1 sequence. Ligation mixtures were transformed to *L. Lactis* NZ9000 using an electroporation protocol described by Holo and Nes (Holo and Nes, 1989) (Figure 2B).

### COS-7 cells transfection.

Two six wells plates with COS-7 cells were transiently transfected with supercoiled plasmid DNA using the GeneJammer transfection reagent (Stratagene) according to the manufacturer's recommendations. Transfection efficiency was normally in the range 5-10%. As a result all cellular preparations contain 90-95% untransfected cells. After two days, one transfected plate was fixed with acetic acid and Ethanol. These cells were used for immunoflourescent staining. The second plate was incubated for three days and scFvSIgA1 protein conditioned medium was aspirated and stored at -20°C until analysis. The rest of the COS-7 cells were washed twice with PBS, pelleted and stored at -20°C until analysis. Later, the proteins from these cells were extracted and stored at -80°C.

### Bacterial strains, growth condition and induction of proteins.

*E. coli* K-12 strain was grown on LB agar and broth. The cells were induced by IPTG to produce the proteins. *L. lactis* strain NZ9000 was cultivated semi-anaerobically at 30°C in M17 broth at pH 6.5, supplemented with 1.0% (w/v) glucose and 5 µg/ml chloramphenicol when carrying pNZmlep or derivatives. For isolation of membrane vesicles, cells were grown to an OD₆₀₀ of 1, after which transcription from the *nisA* promoter was switched on by the addition of 0.2% (v/v) culture supernatant of the nisin A producing strain NZ9700 (de Ruyter et al., 1996). Cells were harvested 1 h after the induction of nisin.

### Membrane vesicle preparation of MOC31pNZmlepSIgA1.

Cell were washed with 100mM KPO4, pH 7.0 and then resuspended in 100mM KPO4, pH 7.0 with 20% glycerol. Cells were recaptured in a bead beater at force 6.5 for 30 seconds for 3 times. Whole cells were removed by centrifugation at 16000 x g for 15 min and membrane fractions were removed by centrifugations at 267000 x g for 30 min. Vesicles were resuspended in 100mM KPO4, pH 7.0 with 20% glycerol and stored in -80 until used.

### Immunofluorescent staining.

Cover slips containing transfected cells were fixed with ethanol and acetic acid. The cells transfected with MOC31-IgA variants were stained with Anti-human IgA-FITC (Dako). MOC31-IgG transfected cells were stained with Alexa fluor 488 goat anti human IgG (H+L) (Molecular Probes). DAPI was used to counter stain the nuclei.

### ELISA.

scFv MOC variant levels in cultured supernatant and in protein extract were measured with an IgA and IgG specific enzyme-linked immunosorbent-assay (ELISA). Duplicate wells of serially diluted supernatants were analyzed as described previously (Guikema et al., 1999).

### Jurkat and Ramos cells FACS

Human cell lines Jurkat and Ramos were stably transfected with EGP-2 as described previously (Bremer et *al.,* 2004) and cultured in RPMI (Invitrogen) media supplemented with 15% fetal calf serum. These cells were incubated for 30 minutes with transfected COS-7 supernatant and protein extract of different MOC variants. After a brief wash the cells were incubated with goat anti human IgA-PE. The FACS analysis was performed on Coulter EpicTM Elite flow cytometer. Data were analyzed by using flowjo software version 5.4.5 according to the manufactures protocol (Tree Star Inc).

### Western blotting

Expression of all MOC31 scFv was confirmed by the Western blotting. The expected size of proteins and percentage of the gel are described in the Table-2 Protein gels were stained with coomassie blue. For immunodetection, samples were separated by reduced SDS-PAGE and electrotransferred to a nitrocellulose membrane. The membrane was blocked with 5% non-fat milk in PBS-T buffer (PBS containing 0.05% Tween 20), and then incubated with different (Table-2) antibodies separately for 2 hours. The membrane was washed three times with PBS-T buffer and then incubated in HRP conjugated secondary antibody for 1 hour. The membrane was washed three times with PBS-T buffer and the expressed proteins were visualized using ECL plus western blotting detection system (Amersham).

### Results

### COS-7 cells transfection.

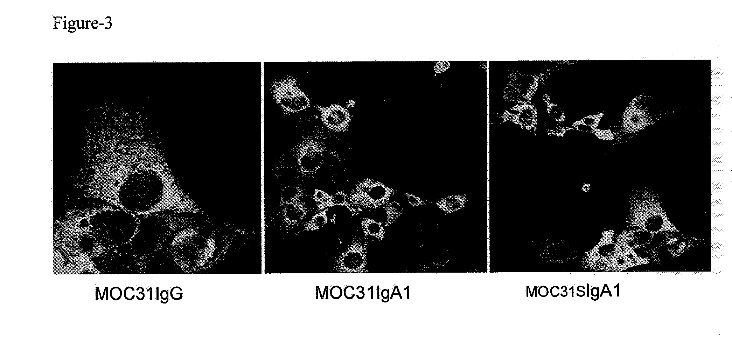

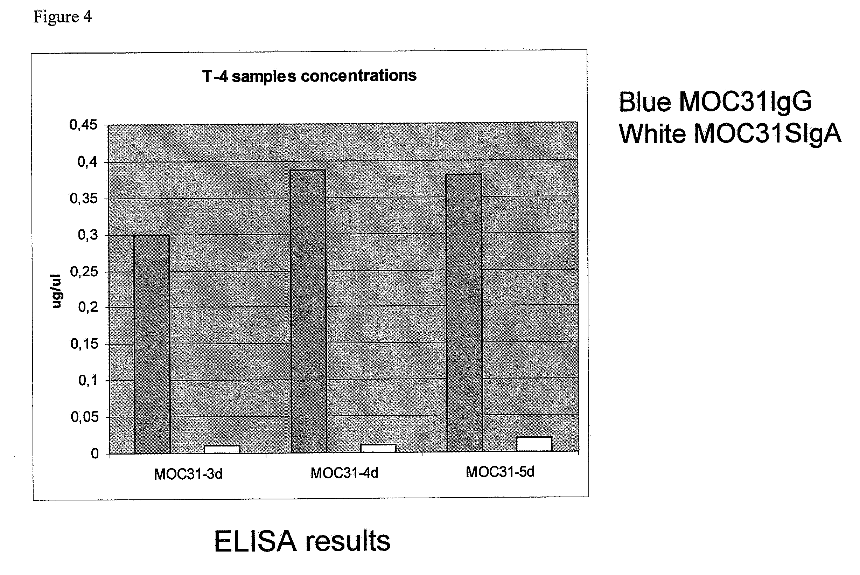

COS-7 cells were transiently transfected with the entire MOC31 variant. Correct orientation of the constructs was validated by this method as well as by sequencing. The transfection efficiency of prokaryotic expression vector MOC31SIgA1 was 10% as shown by immunopositive for anti-human IgA-FITC antibodies (see Figure 3). The presence of secreted scFv MOC31 variants in the transfected COS-7 cells supernatant were analysed by ELISA (see Figure 4). The positive control MOC31IgG1 was produced in higher quantity than the other scFv variants.

### Western blotting

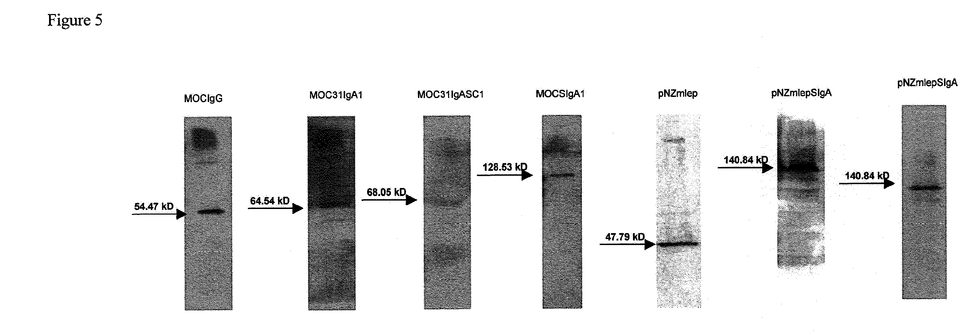

Western blotting was performed on all the MOC variants. MOC31 transfected COS-7 supernatant and COS-7 cell lysate were used for western blotting. All the products gave a expected band with a correct size (see Figure 5). Membrane vesicle was prepared from MOC31pNZmlepSIgA1 followed by western blotting for pNZmlep and pNZmlepSIgA1. Total protein precipitation of *L. lactis* with TCA was performed and used for western blotting. From the above set of experiments we concluded that all the MOC variants were produced, but with a low yield. In order to test the functional activity of these proteins before using in the *in vivo* experiments, following experiment where performed.

### Jurkat and Ramos cells FACS

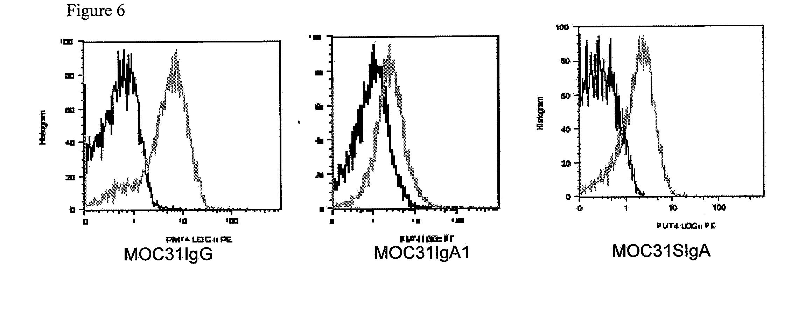

Flow cytometric analysis of the retrovirally transduced cell lines Jurkat.EGP2, Ramos cells confirmed the cell surface expression of EGP2. MOC31-IgA containing supernatant were able to bind to these EGP2 on these cell surfaces and gave strong increase in percentage of functionally active scFv. The binding of MOC31-IgA containing supernatant to the EGP2- expressing Ramos cells proves that IgA is indeed functional (see Figure 6).

### Example 2: Construction of a dual targeting system to apoptosis in colon cancer epithelial cells.

We have successfully engineered a therapeutic proteinaceous substance named BMP-DIgAslac. It consists of:
- MOC31 heavy and light chain variable parts, specific for the colonic epithelial molecule EGP-2
- The complete human IgA constant region (both versions with IgA1 and IgA2 constant regions)
- Domain 1-5 of the human polyIgR
- Two domains 6 of the human polyIgR containing the epithelial endopeptidase sensitive sites
- Bone Morphogenetic Protein-2, able to induce apoptosis in colon cancer cell lines

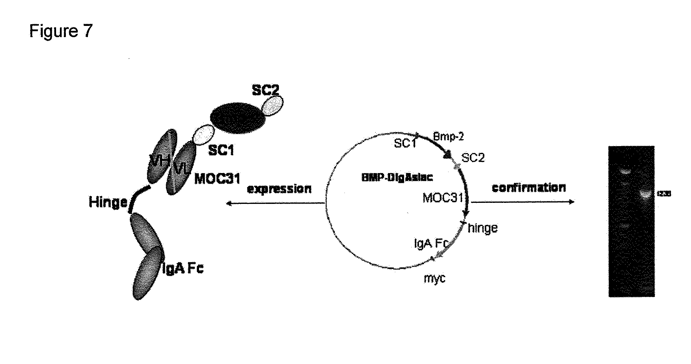

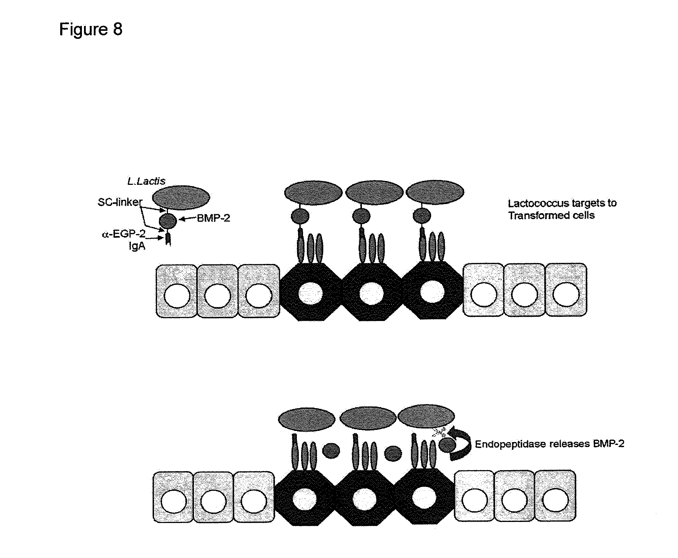

The complete insert was confirmed by sequencing both in eukaryotic and prokaryotic expression vectors (see Figure 7). The protein can be used to target BMP-2 to transformed epithelial cells using *L. lactis* as production and delivery system. Endopeptidase activity of the target cells locally releases the pro-apoptotic BMP-2 molecule (see schematic drawing of Figure 8).

### Example 3: Therapeutic applications of a dual targeting system.

The design of the proteinaceous substance according to the invention is such that both targeting moietie(s) and bioactive polypeptide(s) can be tailor-made for specific purposes. For example, in a vector encoding an exemplary proteinaceous substance, the antibody targeting moiety has been placed in between unique restriction enzymes and heavy and light chain antibody genes can easily be replaced by other desired antibody genes with other specificities. The bioactive polypeptide that is placed in between the two SC-endopeptidase sensitize sites will be cut out specifically by gut epithelial cells. The protein substances are in these examples also referred to as "DIgAslac", which is an abbreviation of 'Digestive IgA secreted by Lactococus" The following gastrointestinal disorders could benefit from the dual targeting system:

### A. Colon cancer

The first prototype DIgAslac has been designed for usage in colon cancer patients. In this prototype the bioactive compound consist of the morphogenetic protein BMP-2. This was chosen for it bioactivity (apoptosis induction) as recombinant protein and its evolutionary conservation. Other possible candidate bioactive compounds are the morphogenetic compound Sonic Hedgehog 2 and the apoptosis inducing compounds TRAIL 3 and Fas 4.
EGP-2 Transgenic mice are available which express human EGP-2 at the colon epithelium. These mice can be used to evaluate targeting of *L. lactis* to the colon epithelium *in vivo.* Since BMP molecules are very conserved among species, effects of released BMP-2 *in vivo* on normal, healthy mice can be evaluated.
Mice can be inoculated with different doses of recombinant *L. lactis* bacteria daily for different time periods. Presence of recombinant *L. lactis* can be evaluated by culturing fecal samples, 16S rRNA specific in-situ hybridization and quantitative PCR. All these assays are available on a routine basis. Biological effects on gut epithelium can be evaluated by histology.

Effects on gut carcinoma can be tested by crossing EGP-2 Tg mice with APC-Min mice. APC-Min mice have a mutation in one of the two alleles of the Adenoma Polyposis Coli (APC) gene. This gene is also affected in the majority of patients with colon carcinoma. Families with a mutation in the APC gene are at very high risk to develop colon carcinoma. The APC-min mice are a good model for colon carcinoma. They develop multiple tumors within three months after birth, mainly within the small intestine.
Since the targeting system also target epithelial cells of the small intestine, these mice provide a good model to test our vector system and the effects on gut carcinomas. Crossed APC-min x EGP-2 mice can be tested for the affected APC gene by tail DNA-PCR. Mice from the same crosses that are negative for the APC gene can serve as internal controls.

Mice can be inoculated with BMP-DIgAslac from 5 weeks of age during four weeks. Around five weeks of age, the APC-Min mice normally develop polyps that can progress into carcinoma. As negative control the parent strain of *L. lactis* can be used. Survival of the animals is an important parameter. Animals can be evaluated histologically for development of polyps and carcinoma.

### B. Chronic inflammatory conditions

The first human phase 1 trial with *Lactococci* secreting (i.e. untargeted) IL-10 has now been successfully completed. Optimizing the effect of IL-10 in inflammatory bowel disease (IBD) can be achieved by introducing IL-10 into a proteinaceous substance according to the invention such that it can be specifically delivered and release at a target cell. It is envisaged that a greater bioavailability of IL-10 can be achieved by specific targeting to epithelial cells. Another promising candidate is a bioactive polypeptide capable of neutralizing the action of TNFα. Anti-TNFα treatment, in particular with Infliximab, seems to be a efficacious treatment modality in patients with IBD. There are however serious drawbacks of the current strategies. The very high costs for continuous intravenous treatment with anti-TNFα antibodies, antibodies to Infliximab (ATI), and risk factors involved with concomitant immunosuppression, such as tuberculosis infections, are reported drawbacks.
An alternative strategy would be an oral administration of probiotic vectors producing continuously anti-TNFα compounds that are targeted towards the affected areas of the gut.
A proteinaceous substance comprising anti-TNFα antibody fragments flanked by protease cleavage sites and epithelial cell targeting means can be used. Different strategies can be followed for development of this new generation of anti-TNF drugs. Firstly, the antibody genes of the single chain IgA antibody can be replaced by antibody genes that are specific for TNF. Secondly, the loading cassette can be inserted with published anti-TNF peptides based on CDR3 homologies 5. Biological activity of the released anti-TNF compounds can be tested by apoptosis induction and cell cycle arrest in human T cell lines expressing mTNF and lamina propria T-lymphocytes from IBD patients.

### C. Immunosuppressed conditions

Patients that are treated with immunosuppressive drugs such as cytostatica (cancer patients) or corticosteroids (patients with autoimmune diseases) have often a depressed mucosal immune system. Supportive therapy can be obtained by non-invasive bacteria expressing IgA antibodies that are relevant in maintaining a homeostasis with the normal gut flora. Opportunistic gastrointestinal infections with otherwise commensal bacteria are often seen.
We have established a library of 200 IgA heavy chain genes from ileal biopsies from normal healthy individuals (Yuvaraj et al., 2006. Human mucosal IgA producing cells are derived from a restricted set of precursors. Manuscript in preparation). We showed that the human ileal IgA repertoire is very restricted. Producing a mix of different DIgAslac (Multi-DIgAslac) with these Ig heavy chain genes can provide a passive IgA vaccine during critical periods.

### D. Gastrointestinal infections.

Both bacterial and viral gastrointestinal infections benefit from the introduction or the induction of IgA antibodies. In mouse rotavirus models we have shown that high-affinity responses are mainly derived from conventional B cells and not from B-1 cells (Kushnir et al., 2001. J.Virol. 75:5482-5490). Furthermore, for complete clearance of the rotavirus T cells are necessary, but IgA antibodies are very important in preventing re-infections. The Ig sequences of specific anti-rotavirus monoclonal antibodies are known and can be introduced into the DIgAslac system. Release of the IgA antibodies can be achieved by the SC system at the site of rotavirus entry in enterocytes or M cells. Also monoclonal antibodies against pathogenic bacteria such as *Salmonella* and *Vibrio cholerae* are described to be effective in clearance of the infection and prevention of reinfection. Introducing of these specific Ig genes into the DIgAslac vector can be used as a quick intervention system during acute gastrointestinal infections.

### E. Oral immunizations.

Oral immunizations are usually hampered by the tolerant status of the mucosal immune system. Production of specific immune responses after oral immunizations were shown to be dependent on IgA-bound antigens. IgA-complexes were internalized into the gut immune system and further processed into the systemic immune system. It can be envisaged that introducing protein vaccine into the SC releasing system of DIgAslac in combination with the MOC31 IgA targeting part towards gut epithelial cells will result in release and IgA-Fc mediated uptake. Subsequent transport to the lymph node can result in good humoral immune responses.

### F. General health improvements.

As stated above we have established a library of 200 IgA heavy chain Ig genes form normal healthy individuals and this library covers a large proportion of the complexity of the IgA repertoire. Multi-DIgAslac can be beneficial for (re-) establishing a good balance with the normal gut flora and can be supportive during and directly after antibiotica treatment. Also during traveling when new environmental challenges are encountered multi-DIgAslac can be beneficial.

### References

1. Almogren,A., Senior,B.W., Kerr,M.A., 2006. A comparison of the binding of secretory component to immunoglobulin A (IgA) in human colostral S-IgAl and S-IgA2. Immunology.
2. Alvarez,R.D., Barnes,M.N., Gomez-Navarro,J., Wang,M., Strong,T.V., Arafat,W., Arani,R.B., Johnson,M.R., Roberts,B.L., Siegal,G.P., Curiel,D.T., 2000. A cancer gene therapy approach utilizing an anti-erbB-2 single-chain antibody-encoding adenovirus (AD21): a phase I trial. Clin. Cancer Res. 6, 3081-3087.
3. Balzar,M., Winter,M.J., de Boer,C.J., Litvinov,S.V., 1999. The biology of the 17-1A antigen (Ep-CAM). J. Mol. Med. 77, 699-712.
4. Bandell,M., Lolkema,J.S., 2000. The conserved C-terminus of the citrate (CitP) and malate (MleP) transporters of lactic acid bacteria is involved in substrate recognition. Biochemistry 2000. Oct. 24. ;39. (42.):13059. -67. 39, 13059-13067.
5. Braat,H., Peppelenbosch,M.P., Hommes,D.W., 2006a. Immunology of Crohn's disease. Ann. N. Y. Acad. Sci. 1072:135-54., 135-154.
6. Braat,H., Rottiers,P., Hommes,D.W., Huyghebaert,N., Remaut,E., Remon, J.P., van Deventer,S.J., Neirynck,S., Peppelenbosch,M.P., Steidler,L., 2006b. A phase I trial with transgenic bacteria expressing interleukin-10 in Crohn's disease. Clin. Gastroenterol. Hepatol. 4, 754-759.
7. Brandtzaeg,P., Farstad,I.N., Johansen,F.E., Morton,H.C., Norderhaug,I.N., Yamanaka,T., 1999. The B-cell system of human mucosae and exocrine glands. Immunol. Rev. 171, 45-87.
8. Bremer,E., Samplonius,D., Kroesen, B.J., van Genne,L., de Leij,L., Helfrich,W., 2004. Exceptionally potent anti-tumor bystander activity of an scFv:sTRAIL fusion protein with specificity for EGP2 toward target antigen-negative tumor cells. Neoplasia. 6, 636-645.
9. Bremer,E., Ten Cate,B., Samplonius,D.F., de Leij,L.F., Helfrich,W., 2005. CD7-restricted activation of Fas-mediated apoptosis: a novel therapeutic approach for acute T-cell leukemia. Blood...
10. de Ruyter,P.G., Kuipers, O.P., de Vos,W.M., 1996. Controlled gene expression systems for Lactococcus lactis with the food-grade inducer nisin. Appl. Environ. Microbiol. 62, 3662-3667.
11. Elm,C., Braathen,R., Bergmann,S., Frank,R., Vaerman,J.P., Kaetzel,C.S., Chhatwal,G.S., Johansen,F.E., Hammerschmidt,S., 2004. Ectodomains 3 and 4 of human polymeric Immunoglobulin receptor (hpIgR) mediate invasion of Streptococcus pneumoniae into the epithelium. J. Biol. Chem. 2004. Feb. 20.;279. (8.):6296. -304. Epub. 2003. Dec. 3. 279, 6296-6304.
12. Glennie,M.J., Johnson, P.W., 2000. Clinical trials of antibody therapy. Immunol. Today. 21, 403-410.
13. Gomes,M.M., Herr,A.B., 2006. IgA and IgA-specific receptors in human disease: structural and functional insights into pathogenesis and therapeutic potential. Springer Semin. Immunopathol...
14. Guikema,J.E., Vellenga,E., Veeneman,J.M., Hovenga,S., Bakkus,M.H., Klip,H., Bos,N.A., 1999. Multiple myeloma related cells in patients undergoing autologous peripheral blood stem cell transplantation. Br. J. Haematol. 104, 748-754.
15. Helfrich,W., Kroesen,B.J., Roovers,R.C., Westers,L., Molema,G., Hoogenboom,H.R., de Leij,L., 1998. Construction and characterization of a bispecific diabody for retargeting T cells to human carcinomas. Int. J. Cancer. 76, 232-239.
16. Holo,H., Nes,I.F., 1989. High-Frequency Transformation, by Electroporation, of Lactococcus lactis subsp. cremoris Grown with Glycine in Osmotically Stabilized Media. Appl. Environ. Microbiol. 55, 3119-3123.
17. Huls,G., Heijnen,I.A., Cuomo,E., van der,L.J., Boel,E., Van De Winkel,J.G., Logtenberg,T., 1999. Antitumor immune effector mechanisms recruited by phage display-derived fully human IgG1 and IgA1 monoclonal antibodies. Cancer Res. 59, 5778-5784.
18. Johansen,F.E., Brandtzaeg,P., 2004. Transcriptional regulation of the mucosal IgA system. Trends Immunol. 2004. Mar. ;25. (3):150. -7. 25, 150-157.
19. Kanamaru,Y., Tamouza,H., Pfirsch,S., Elmehdi,D., Guerin-Marchand,C., Pretolani,M., Blank,U., Monteiro,R.C., 2006. IgA Fc receptor I signals apoptosis through the FcR{gamma}ITAM and affects tumor growth. Blood...
20. Moldenhauer,G., Momburg,F., Moller,P., Schwartz,R., Hammerling,G.J., 1987. Epithelium-specific surface glycoprotein of Mr 34,000 is a widely distributed human carcinoma marker. Br. J. Cancer. 56, 714-721.
21. Ragnhammar,P., Fagerberg,J., Frodin,J.E., Hjelm,A.L., Lindemalm,C., Magnusson,I., Masucci,G., Mellstedt,H., 1993a. Effect of monoclonal antibody 17-1A and GM-CSF in patients with advanced colorectal carcinoma--long-lasting, complete remissions can be induced. Int. J. Cancer. 53, 751-758.
22. Ragnhammar,P., Magnusson,I., Masucci,G., Mellstedt,H., 1993b. The therapeutic use of the unconjugated monoclonal antibodies (MAb) 17-1A in combination with GM-CSF in the treatment of colorectal carcinoma (CRC). Med. Oncol. Tumor Pharmacother. 10, 61-70.
23. Riethmuller,G., Holz,E., Schlimok,G., Schmiegel,W., Raab,R., Hoffken,K., Gruber,R., Funke,I., Pichlmaier,H., Hirche,H., Buggisch,P., Witte,J., Pichlmayr,R., 1998. Monoclonal antibody therapy for resected Dukes' C colorectal cancer: seven-year outcome of a multicenter randomized trial. J. Clin. Oncol. 16, 1788-1794.
24. Schaedel,O., Reiter,Y., 2006. Antibodies and their fragments as anti-cancer agents. Curr. Pharm. Des. 12, 363-378.
25. Schjerven,H., Brandtzaeg,P., Johansen,F.E., 2001. A novel NF-kappa B/Rel site in intron 1 cooperates with proximal promoter elements to mediate TNF-alpha-induced transcription of the human polymeric Ig receptor. J. Immunol. 2001. Dec. 1;167. (11):6412. -20. 167, 6412-6420.
26. Valerius,T., Stockmeyer,B., van Spriel,A.B., Graziano,R.F., van den Herik-Oudijk IE, Repp,R., Deo,Y.M., Lund,J., Kalden,J.R., Gramatzki,M., Van De Winkel,J.G., 1997. FcalphaRI (CD89) as a novel trigger molecule for bispecific antibody therapy. Blood. 90, 4485-4492.
27. Van den Brande,J.M., Koehler,T., Zelinkova,Z., Bennink,R.J., Te Velde,A.A., Ten Kate,F., van Deventer,S.J., Peppelenbosch,M.P., Hommes,D.W., 2006. Prediction of anti-TNF clinical efficacy by real-time visualisation of apoptosis in patients with Crohn's disease. Gut. ..
28. Vogelstein,B., Kinzler,K.W., 2004. Cancer genes and the pathways they control. Nat. Med. 10, 789-799.
29. Withoff,S., Helfrich,W., de Leij,L.F., Molema,G., 2001. Bi-specific antibody therapy for the treatment of cancer. Curr. Opin. Mol. Ther. 3, 53-62.
30. Yuvaraj,S., Peppelenbosch,M.P., Bos,N.A., 2007. Transgenic probiotica as drug delivery systems: the golden bullet? Expert. Opin. Drug Deliv. 4, 1-3.

## Claims

1. A therapeutic proteinaceous substance comprising at least one targeting moiety capable of binding to a target cell surface molecule, and at least one bioactive polypeptide, wherein said bioactive polypeptide is flanked on both sides by at least one cleavage site for an endopeptidase activity associated with said target cell.

2. Proteinaceous substance according to claim 1, wherein said target cell is an epithelial cell, preferably intestinal epithelial cell.

3. Proteinaceous substance according to claim 1 or 2, wherein said cleavage site is a cleavage site for epithelial endopeptidase, preferably wherein said cleavage site is a cleavage site of the human PolyIgR or a functional equivalent thereof.

4. Proteinaceous substance according to claim 3, wherein the cleavage site corresponds to a cleavage site in domain 6 of the human PolyIgR.

5. Proteinaceous substance according to any one of claims 1 to 4, furthermore comprising at least one stabilizing domain capable of conferring stability of the substance in the gut.

6. Proteinaceous substance according to claim 5, wherein said stabilizing domain is an IgG constant region (IgG Fc) or an IgA constant region (IgA Fc), preferably IgA Fc, more preferably IgA Fc complexed to domains 1-5 of the human polyIgR.

7. Proteinaceous substance according to any one of the above claims, wherein said at least one targeting moiety is an antibody or a functional fragment thereof, preferably a single chain variable antibody fragment (scFv).

8. Proteinaceous substance according to any one of the above claims, wherein said target cell surface molecule is selected from the group consisting of ligands that are overexpressed or exclusively expressed on diseased cells, including tumor cells and inflamed cells, and stress induced (glyco)proteins.

9. Proteinaceous substance according to claim 8, wherein said target cell surface molecule is selected from the group consisting of EGP-2, EGFR-3 and proteins encoded by non-classical MHC class I chain-related (MIC) genes MICA and MICB.

10. Proteinaceous substance according to any one of the above claims, wherein said at least one bioactive polypeptide is selected from the group consisting of antibodies, cytokines, growth factors, differentiation factors, morphogenic polypeptides, apoptosis-inducing polypeptides and immunogenic peptides.

11. Proteinaceous substance according to claim 10, wherein said bioactive polypeptide is selected from the group consisting of IgA antibodies, IgG antibodies, bone morphogenetic protein (BMP), hedgehog, TGF-β family members, TNF-α, IFNγ, and ligands for death receptors including sTRIAL and FasL.

12. A nucleic acid sequence encoding a proteinaceous substance according to any one of claims 1 to 11.

13. Nucleic acid sequence according to claim 12, cloned in frame with a nucleic acid sequence encoding a microbial membrane anchoring domain, preferably encoding a bacterial transmembrane protein, such as an ABC transporter.

14. An expression vector comprising at least one nucleic acid sequence of claim 12 or 13.

15. Non-invasive or non-pathogenic microbial host cell provided with at least one vector according to claim 14 and capable of expressing the at least one encoded proteinaceous substance.

16. Host cell according to claim 15, selected from the group consisting of non-colonising bacteria, food-grade bacteria, Gram-positive bacteria, attenuated Gram-positive pathogenic bacteria, and yeast cells.

17. Host cell according to claim 16, selected from *Listeria innocua, Staphylococcus xylosus* and *Lactococcus* species, preferably *Lactococcus lactis.*

18. A composition, preferably a food or pharmaceutical composition, comprising a proteinaceous substance according to any one of claims 1 to 11 or a host cell according to any one of claims 15 to 17, and a suitable carrier.

19. Use of a proteinaceous substance according to any one of claims 1 to 11 or a host cell according to any one of claims 15 to 17 for the manufacture of a medicament for the treatment or profylaxis of an intestinal disease or disorder.

20. Use according to claim 19, wherein said disease is selected from the group consisting of cancer, including polyposis and colon carcinoma, (chronic) inflammatory intestinal conditions such as inflammatory bowel disease, coeliac disaese, and gastrointestinal infections.
